# EUROPÄISCHE PATENTSCHRIFT

(11) **EP 0 726 248 B1**
(45) Veröffentlichungstag und Bekanntmachung des Hinweises auf die Patenterteilung: **15.04.1998**
(21) Anmeldenummer: 96100858.8
(22) Anmeldetag: 23.01.1996
(51) Int. Cl.: C07C 235/16, C07C 231/12

(54) **Verfahren zur Herstellung von O-Acyloxycarbonsäureaniliden**
Process for the preparation of anilides of O-acyloxycarboxylic acids
Procédé de préparation d'anilides des acides O-acyloxycarboxyliques

(30) Priorität: 09.02.1995 DE 19504225
(43) Veröffentlichungstag der Anmeldung: 14.08.1996
(73) Patentinhaber: Clariant GmbH, 65929 Frankfurt am Main (DE)
(72) Erfinder: Dierdorf, Andreas, Dr., D-60529 Frankfurt (DE); Papenfuhs, Theodor, Dr., D-60433 Frankfurt (DE); Planker, Siegfried, Dr., D-61462 Königstein (DE)

(56) Entgegenhaltungen:
- EP-A- 0 014 409
- DE-A- 2 201 432
- DE-A- 3 038 598
- H. PIELARTZIK ET AL. 'HOUBEN-WEYL "Methoden der organischen Chemie, Band E5, 1985' , GEORG THIEME VERLAG , STUTTGART . NEW YORK * Seite 684 - Seite 690 *

## Beschreibung

Die vorliegende Erfindung betrifft ein gegenüber dem Stand der Technik verbessertes Verfahren zur Herstellung von O-Acyloxycarbonsäureaniliden.

O-Acyloxycarbonsäureanilide, insbesondere Acetoxyessigsäureanilide, stellen interessante Vorprodukte zur Herstellung von Glykolsäureaniliden dar. Glykolsäureanilide wiederum finden als wichtige Ausgangsstoffe sowohl bei der Herstellung von Herbiziden (EP-A-300 344) und pharmazeutischen Wirkstoffen (EP-A-284 338, EP-A-363 284) als auch bei der Herstellung von Fungiziden (US 4 440 780) Anwendung.

Aufgrund der Bedeutung dieser Stoffgruppe hat es in der Vergangenheit nicht an Versuchen gefehlt, Hydroxycarbonsäureamide und insbesondere Hydroxycarbonsäureanilide, beispielsweise Glykolsäureanilide, auf unterschiedlichen Wegen zugänglich zu machen. Einer dieser Wege besteht darin, ein α-Chlorcarbonsäureamid mit einem Alkaliformiat oder Alkaliacetat in Gegenwart eines quartären Ammoniumsalzes umzusetzen.

Die DE-OS 30 38 598 beschreibt ein Verfahren zur Herstellung von α-Hydroxycarbonsäureamiden durch Umesterung von Acetoxycarbonsäureamiden mittels Alkoholen in Gegenwart katalytischer Mengen eines Alkali- oder Erdalkalihydroxids oder -carbonats. Man setzt die entsprechenden α-Halogencarbonsäureamide mit einem Alkali- oder Erdalkaliacetat in Gegenwart eines quartären Ammoniumsalzes und gegebenenfalls unter Verwendung eines Verdünnungsmittels zu den entsprechenden α-Acetoxycarbonsäureamiden um, die anschließend umgesetzt werden.

Die US 4 334 073 betrifft ein Verfahren zur Herstellung von α-Hydroxycarbonsäureamiden, wobei zunächst ein α-Halogencarbonsäureamid mit einem Alkaliacetat oder Erdalkaliacetat in Gegenwart eines quartären Ammoniumsalzes und eines Lösungsmittels umgesetzt wird und anschließend das erhaltene α-Acetoxycarbonsäureamid zu dem entsprechenden α-Hydroxycarbonsäureamid decarboxyliert wird.

Von Nachteil ist, daß die Herstellung der Acetoxycarbonsäureamide unter Verwendung quartärer Ammoniumsalze erfolgt. Es ist nämlich bekannt, daß derartige quartäre Ammoniumsalze insbesondere im Abwasser unerwünscht sind, da sie zu Problemen in der Abwasseraufbereitung führen.

Ein weiterer Nachteil ist, daß der Einsatz eines quartären Ammoniumsalzes keineswegs eine Umsetzung garantiert, die in einer akzeptablen Reaktionszeit zu einer guten Ausbeute (definiert als Umsatz x Selektivität) führt. Dies belegt der entsprechend zur US 4 334 073 durchgeführte Vergleichsversuch.

Im Hinblick auf die Bedeutung von O-Acyloxycarbonsäureaniliden, insbesondere Acetoxyessigsäureaniliden, als Vorprodukte zur Herstellung von Glykolsäureaniliden stellt es eine interessante Aufgabe dar, ein Verfahren zur Herstellung von O-Acyloxycarbonsäureaniliden bereitzustellen, das einerseits die Nachteile der vorstehend genannten Verfahren vermeidet, sich andererseits auf einfache Weise und unter Verwendung leicht zugänglicher Ausgangsstoffe durchführen läßt und zudem die gewünschten O-Acyloxycarbonsäureanilide sowohl in guter Ausbeute als auch in hoher Reinheit liefert.

Gelöst wird diese Aufgabe durch ein Verfahren zur Herstellung von O-Acyloxycarbonsäureaniliden der allgemeinen Formel (I) 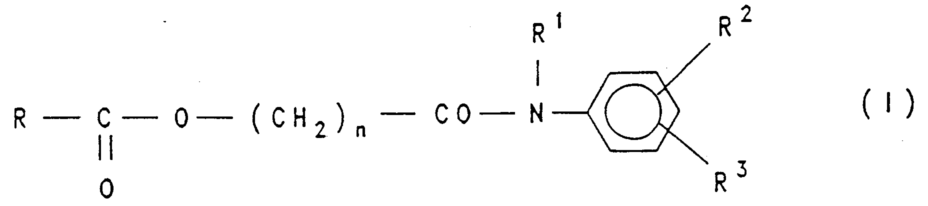 worin R für Wasserstoff oder einen Rest mit 1 bis 6 Kohlenstoffatomen steht, n eine ganze Zahl von 1 bis 10 ist, R¹ Wasserstoff oder einen Alkylrest mit 1 bis 12 Kohlenstoffatomen bedeutet, R² und R³ gleich oder verschieden sind und für Wasserstoff, einen Alkylrest mit 1 bis 12 Kohlenstoffatomen, einen Arylrest mit 6 bis 12 Kohlenstoffatomen, einen Cycloalkylrest mit 5 bis 12 Kohlenstoffatomen im Ring, einen Alkenyl- oder Alkinylrest mit 3 bis 12 Kohlenstoffatomen, für NO₂, F, Cl, Br oder CN stehen. Es ist dadurch gekennzeichnet, daß man ein Chlorcarbonsäureanilid der allgemeinen Formel (II) 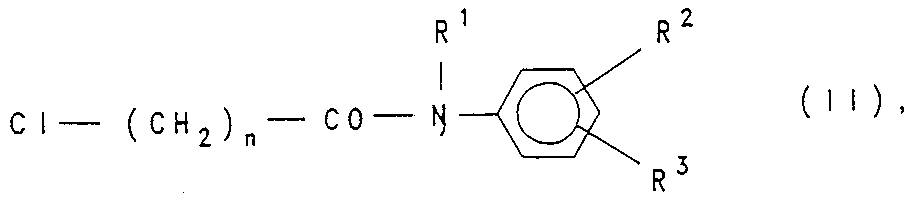 worin n, R¹, R² und R³ die obengenannte Bedeutung besitzen, mit einem Alkalimetallcarboxylat der allgemeinen Formel (III)

R-COOMe (III),

worin R die obengenannte Bedeutung hat und Me für ein Alkalimetall steht, in Anwesenheit eines inerten Lösungsmittels und einer Carbonsäure mit 1 bis 6 Kohlenstoffatomen bei 50 bis 200°C umsetzt.

Das erfindungsgemäße Verfahren weist mehrere Vorteile auf. Zum einen ist es nicht auf die Herstellung von Acetoxyessigsäureaniliden beschränkt, sondern eignet sich generell für die Herstellung von O-Acyloxycarbonsäureaniliden entsprechend der Umsetzung des Chlorcarbonsäureanilid der Formel (II) mit dem Alkalimetallcarboxylat der Formel (III). Zum anderen handelt es sich bei dem Alkalimetallcarboxylat, dem inerten Lösungsmittel ebenso wie bei der Carbonsäure um leicht zugängliche Ausgangsstoffe, die auch in technischen Mengen zur Verfügung gestellt werden können. Das Verfahren läßt sich auf einfache Weise, also ohne einen hohen technischen Aufwand, realisieren und es führt bei hohen Umsätzen zu guten Ausbeuten. Das gewünschte Endprodukt fällt dabei in sehr hoher Reinheit an.

Zudem kann auf die Verwendung von quartären Ammoniumsalzen generell verzichtet werden. Da das Verfahren in Abwesenheit quartärer Ammoniumsalze abläuft, ergeben sich auch nicht die vorstehend geschilderten Probleme bei der Abwasseraufarbeitung.

Wie aus den vorangegangenen Ausführungen hervorgeht, eignet sich das erfindungsgemäße Verfahren für die Umsetzung einer größeren Zahl verschiedener Chlorcarbonsäureanilide. So lassen sich Chlorcarbonsäureanilide der Formel (II), worin n für eine ganze Zahl von 1 bis 4, insbesondere 1 bis 2, bevorzugt für 1 steht, mit gutem Erfolg einsetzen.

Von Interesse sind Chlorcarbonsäureanilide der Formel (II), worin R¹ für Wasserstoff oder einen Alkylrest mit 1 bis 4 Kohlenstoffatomen steht und solche, worin R² und R³ gleich oder verschieden sind und für Wasserstoff, einen Alkylrest mit 1 bis 4 Kohlenstoffatomen, für NO₂, F, Cl, Br oder CN stehen und insbesondere R² Wasserstoff ist und R³ für einen Alkyrest mit 1 bis 4 Kohlenstoffatomen, für NO₂, F, Cl, Br oder CN steht.

In das Verfahren läßt sich eine größere Zahl verschiedener Alkalimetallcarboxylate einsetzen. Insbesondere lassen sich Alkalimetallcarboxylate der Formel (III), worin R für Wasserstoff oder einen aliphatischen Rest mit 1 bis 5 Kohlenstoffatomen, insbesondere für einen aliphatischen Rest mit 1 bis 4 Kohlenstoffatomen und, unabhängig hiervon, Me für Na oder K, insbesondere Na steht, einsetzen.

Man kann das Chlorcarbonsäureanilid und das Alkalimetallcarboxylat in einem weiten Molverhältnis zur Umsetzung bringen. Üblicherweise wendet man das Chlorcarbonsäureanilid und das Alkalimetallcarboxylat im Molverhältnis 1:1 bis 1:5, insbesondere 1:1 bis 1:2 an.

Als inertes Lösungsmittel eignen sich aromatische oder aliphatische Kohlenwasserstoffe, beispielsweise Toluol, o-Xylol, m-Xylol, p-Xylol, Gemische isomerer Xylole, Ethylbenzol, Mesitylen, Chlorbenzol, Dichlorbenzol, Chlortoluol, Cyclohexan, Cumol, Decalin oder Gemische dieser Lösungsmittel, insbesondere Gemische isomerere Xylole und Mesitylen.

Es hat sich bewährt, als Carbonsäure eine aliphatische Carbonsäure mit 1 bis 6, insbesondere 2 bis 5 Kohlenstoffatomen zu verwenden.
Besonders bewährt haben sich Essigsäure und Propionsäure.
Es empfiehlt sich, eine Carbonsäure, die die gleiche Zahl Kohlenstoffatome wie das jeweils verwendete Alkalimetallcarboxylat besitzt, zu gebrauchen. Man kann jedoch auch eine Carbonsäure und ein Alkalimetallcarboxylat mit unterschiedlicher Zahl von Kohlenstoffatomen verwenden, erhält dabei Produkte der Formel (I), in denen R einerseits aus der Carbonsäure andererseits aus dem Carboxylat entstammt.

Üblicherweise verwendet man das Alkalimetallcarboxylat und die Carbonsäure im Molverhältnis 1:0,5 bis 1:5, insbesondere 1:0,7 bis 1:2. Man kann auch unterhalb oder oberhalb dieses Molverhältnisses arbeiten, muß dafür aber unter Umständen höhere Reaktionszeiten oder etwas niedrigere Ausbeuten in Kauf nehmen.

In vielen Fällen hat es sich bewährt, inertes Lösungsmittel zu Carbonsäure im Gewichtsverhältnis 1:0,02 bis 1:0,25, insbesondere 1:0,03 bis 1:0,15, bevorzugt 1:0,04 bis 1:0,1 einzusetzen.

Es hat sich für eine Vielzahl von Umsetzungen als ausreichend erwiesen, die Reaktion bei 70 bis 170, insbesondere 90 bis 140°C ablaufen zu lassen.

Das bei der Umsetzung entstehende Alkalichlorid wird nach Beendigung der Reaktion, beispielsweise durch Filtration und/oder Extraktion, zum Beispiel in Form einer wäßrigen Lösung, abgetrennt. Falls gewünscht, läßt sich das inerte Lösungsmittel destillativ abtrennen und, gegebenenfalls zusammen mit abgetrennter Carbonsäure, wieder in die Reaktion einsetzen.

Die nachfolgenden Beispiele belegen die Erfindung, ohne sie zu beschränken.

### Experimenteller Teil

### Beispiel 1

In einem 250 ml Kolben, bestückt mit Rückflußkühler, Rührer und Thermometer, werden 23,0 g (0,1 mol) Chloressigsäure-N-isopropyl-(4-fluoranilid), 9,0 g (0,11 mol) wasserfreies Natriumacetat, 5 ml Eisessig und 100 ml Xylol unter Rühren auf Rückflußtemperatur erhitzt. Der Reaktionsverlauf wird mittels gaschromatographischer Analyse überwacht.
Nach Beendigung der Reaktion wird das gebildete Natriumchlorid abfiltriert, das Lösungsmittel abdestilliert und der Rückstand durch Umkristallisation oder Destillation gereinigt.
Die Ergebnisse sind der nachfolgenden Tabelle 1 zu entnehmen.

**Tabelle 1**

| Reaktionszeit (Stunden) | Ausbeute (%) * |
|---|---|
| 3 | 75,07 % |
| 4 | 79,7 % |
| 6,75 | 89,6 % |

| | |
|---|---|
| * Ausbeute gaschromatographisch bestimmt | |

### Vergleichsbeispiel 1 (gemäß US 4 334 073)

In einem Kolben, bestückt mit Rückflußkühler, Rührer und Thermometer, werden 229,5 g (1 mol) Chloressigsäure-N-isopropyl-(4-fluoranilid) in 320 ml Toluol, 82 g (1 mol) wasserfreies Natriumacetat und 0,5 g Benzyltrimethylammoniumchlorid unter Rühren 6 Stunden auf 115 bis 120°C erhitzt. Der Reaktionsverlauf wird mittels gaschromatographischer Analyse überwacht.
Die Ergebnisse sind der nachfolgenden Tabelle 2 zu entnehmen.

**Tabelle 2**

| Reaktionszeit (Stunden) | Ausbeute (%) * |
|---|---|
| 1 | 2,9 |
| 2 | 4,5 |
| 3 | 5,7 |
| 4 | 6,8 |
| 6 | 10,3 |

| | |
|---|---|
| * Ausbeute gaschromatographisch ermittelt | |

### Beispiel 2a und 2b

### Einfluß des Verhältnisses Natriumacetat zu Essigsäure

Es werden zwei bis auf die eingesetzte Menge Essigsäure identische Ansätze gefahren, um den Einfluß des Verhältnisses Natriumacetat zu Essigsäure auf die Reaktionszeit und die Ausbeute zu untersuchen.

### Beispiel 2a

In einem Kolben, bestückt mit Rückflußkühler, Rührer und Thermometer, werden 23,0 g (0,1 mol) N-Chloracetyl-N-isopropyl-(4-fluoranilin) mit 9,0 g (0,11 mol) wasserfreiem Natriumacetat, 6,6 g (0,11 mol) Eisessig und 100 ml Xylol unter Rühren auf Rückflußtemperatur erhitzt. Der Reaktionsverlauf wird mittels gaschromatographischer Analyse überwacht.
Die Ergebnisse sind der nachfolgenden Tabelle 3 zu entnehmen.

### Beispiel 2b

Es wird wie in Beispiel 2a beschrieben, gearbeitet, jedoch werden statt 6,6 g Eisessig nunmehr 13,2 g (0,22 mol) Eisessig eingesetzt.

Die Ergebnisse sind der nachfolgenden Tabelle 3 zu entnehmen.

**Tabelle 3**

| Reaktionszeit (Stunden) | Beispiel 2a Ausbeute (%)* | Beispiel 2b Ausbeute (%)* |
|---|---|---|
| 1 | 32 | 32 |
| 2 | 54 | 54 |
| 3 | 66 | 65 |
| 4 | 75 | 75 |
| 5 | 83 | 82 |
| 6 | 87 | 83 |
| 7 | 89 | 84 |
| 8 | 91 | 86 |
| 9 | 91 | 86 |

| | | |
|---|---|---|
| * Ausbeute gaschromatographisch ermittelt | | |

### Beispiel 3

In einem Kolben werden 57,4 g (0,25 mol) N-Chloracetyl-N-isopropyl-(4-fluoranilin), 20,5 g (0,25 mol) wasserfreies Natriumacetat, 12,5 ml Eisessig und 80 ml Xylol unter Rühren auf Rückflußtemperatur erhitzt.
Der Reaktionsverlauf wird mittels gaschromatographischer Analyse überwacht.
Die Ergebnisse sind der nachfolgenden Tabelle 4 zu entnehmen.

### Vergleichsbeispiel 2 (ohne Zusatz von Eisessig)

Es wird, wie in Beispiel 3 angegeben, gearbeitet, jedoch kein Eisessig zugesetzt. Die Reaktion wird mittels gaschromatographischer Analyse überwacht.

Die Ergebnisse sind der nachfolgenden Tabelle 4 zu entnehmen.

**Tabelle 4**

| Reaktionszeit (Stunden) | Vergleichsbeispiel 2 Ausbeute * | Beispiel 3 |
|---|---|---|
| 1 | 0,5 | 33 % |
| 2 | 1 | 54 % |
| 3 | 1,5 | 67 % |
| 4 | 2 | 76 % |
| 5 | 2,5 | 82 % |
| 6 | 3 | 87 % |
| 7 | 3,5 | 89 % |
| 8 | 4 | 91 % |
| 9 | 4,5 | 92 % |
| 10 | 5 | 93 % |

| | | |
|---|---|---|
| * Ausbeute gaschromatographisch ermittelt | | |

### Beispiel 4 und Vergleichsbeispiel 3

### Umsetzung von N-Chloracetyl-N-methylanilin

### Beispiel 4

In einem Kolben, bestückt mit Rückflußkühler, Rührer und Thermometer, werden 18,4 g (0,1 mol) N-Chloracetyl-N-methylanilin, 9,0 g (0,11 mol) Natriumacetat, 6,6 g (0,11 mol) Eisessig und 100 ml Xylol unter Rühren auf Rückflußtemperatur erhitzt. Der Reaktionsverlauf wird mittels gaschromatographischer Analyse überwacht.
Die Ergebnisse sind der nachfolgenden Tabelle 5 zu entnehmen.

### Vergleichsbeispiel 3

Es wird, wie in Beispiel 4 angegeben, gearbeitet, jedoch kein Eisessig zugesetzt. Der Reaktionsverlauf wird mittels gaschromatographischer Analyse überwacht.
Die Ergebnisse sind der nachfolgenden Tabelle 5 zu entnehmen.

**Tabelle 5**

| Reaktionszeit (Stunden) | Vergleichsbeispiel 3 Ausbeute (%) * | Beispiel 4 Ausbeute (%) * |
|---|---|---|
| 1 | 1 | 37 |
| 2 | 2 | 60 |
| 3 | 3 | 72 |
| 4 | 4 | 82 |
| 5 | 5 | 87 |
| 6 | 6 | 91 |
| 7 | 7 | 93 |
| 8 | 8 | 95 |
| 9 | 9 | 98 |

| | | |
|---|---|---|
| * Ausbeute gaschromatographisch ermittelt | | |

### Vergleichsbeispiel 4

Es wird, wie in Beispiel 4 angegeben, gearbeitet, statt 6,6 g (0,11 mol) Eisessig werden jedoch 12,9 g einer 30 Gew.-%igen wäßrigen HCl-Lösung (entsprechend 0,11 mol HCl) zugesetzt. Nach 3,5stündigem Erhitzen auf Rückflußtemperatur können lediglich Spuren des gewünschten Wertprodukts (< 0,2 %) nachgewiesen werden.

### Beispiel 5

In einem Kolben, bestückt mit Rückflußkühler, Rührer und Thermometer, werden 23,0 g (0,1 mol) N-Chloracetyl-N-isopropyl-(4-fluoranilin), 10,6 g (0,11 mol) Natriumpropionat, 8,2 g (0,11 mol) Propionsäure und 100 ml Xylol unter Rühren auf Rückflußtemperatur erhitzt. Der Reaktionsverlauf wird mittels gaschromatographischer Analyse überwacht. Die Ergebnisse sind der nachfolgenden Tabelle 6 zu entnehmen. Im Vergleich zum System Natriumacetat/Essigsäure ist eine signifikante Steigerung der Reaktionsgeschwindigkeit zu beobachten. Die Umsetzung ist nämlich bereits nach 1 Stunde fast abgeschlossen.

### Vergleichsbeispiel 5

Es wird, wie in Beispiel 5 angegeben gearbeitet, jedoch keine Propionsäure zugesetzt. Die Ergebnisse sind der nachfolgenden Tabelle 6 zu entnehmen.

**Tabelle 6**

| Reaktionszeit (Stunden) | Vergleichbeispiel 5 Ausbeute (%) * | Beispiel 5 Ausbeute (%) * |
|---|---|---|
| 1 | 17 | 94 |
| 2 | 28 | 97 |
| 3 | 37 | 98 |
| 4 | 45 | 98 |
| 5 | 53 | 98 |
| 6 | 59 | 98 |
| 7 | 64 | 98 |
| 8 | 68 | 98 |
| 9 | 72 | 98 |
| 10 | 76 | 98 |

| | | |
|---|---|---|
| * Ausbeute gaschromatographisch ermittelt | | |

## Patentansprüche

1. Verfahren zur Herstellung von O-Acyloxycarbonsäureaniliden der allgemeinen Formel (I) 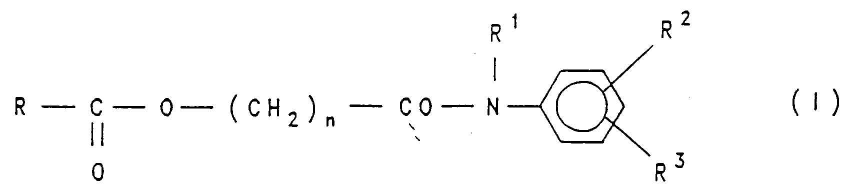 worin R für Wasserstoff oder einen Rest mit 1 bis 6 Kohlenstoffatomen steht, n eine ganze Zahl von 1 bis 10 ist, R¹ Wasserstoff oder einen Alkylrest mit 1 bis 12 Kohlenstoffatomen bedeutet, R² und R³ gleich oder verschieden sind und für Wasserstoff, einen Alkylrest mit 1 bis 12 Kohlenstoffatomen, einen Arylrest mit 6 bis 12 Kohlenstoffatomen, einen Cycloalkylrest mit 5 bis 12 Kohlenstoffatomen im Ring, einen Alkenyl- oder Alkinylrest mit 3 bis 12 Kohlenstoffatomen, für NO₂, F, Cl, Br oder CN stehen, dadurch gekennzeichnet, daß man ein Chlorcarbonsäureanilid der allgemeinen Formel (II) 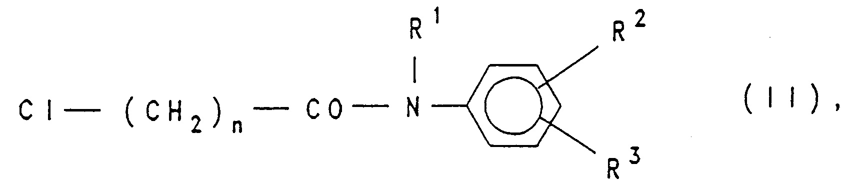 worin n, R¹, R² und R³ die obengenannte Bedeutung besitzen, mit einem Alkalimetallcarboxylat der allgemeinen Formel (III)
R-COOMe (III),
worin R die obengenannte Bedeutung hat und Me für ein Alkalimetall steht, in Anwesenheit eines inerten Lösungsmittels und einer Carbonsäure mit 1 bis 6 Kohlenstoffatomen bei 50 bis 200°C umsetzt.

2. Verfahren nach Anspruch 1, dadurch gekennzeichnet, daß man ein Chlorcarbonsäureanilid der Formel (II), worin n für eine ganze Zahl von 1 bis 4, insbesondere 1 bis 2 steht, einsetzt.

3. Verfahren nach Anspruch 1 oder 2, dadurch gekennzeichnet, daß man ein Chlorcarbonsäureanilid, worin n gleich 1 ist, einsetzt.

4. Verfahren nach einem oder mehreren der Ansprüche 1 bis 3, dadurch gekennzeichnet, daß man ein Chlorcarbonsäureanilid, worin R¹ für Wasserstoff oder einen Alkylrest mit 1 bis 4 Kohlenstoffatomen steht, einsetzt.

5. Verfahren nach einem oder mehreren der Ansprüche 1 bis 4, dadurch gekennzeichnet, daß man ein Chlorcarbonsäureanilid, worin R² und R³ gleich oder verschieden sind und für Wasserstoff, einen Alkylrest mit 1 bis 4 Kohlenstoffatomen, für NO₂, F, Cl, Br oder CN stehen, einsetzt.

6. Verfahren nach einem oder mehreren der Ansprüche 1 bis 5, dadurch gekennzeichnet, daß man ein Chlorcarbonsäureanilid, worin R² Wasserstoff ist und R³ für einen Alkylrest mit 1 bis 4 Kohlenstoffatomen, für NO₂, F, Cl, Br oder CN steht, einsetzt.

7. Verfahren nach einem oder mehreren der Ansprüche 1 bis 6, dadurch gekennzeichnet, daß man ein Alkalimetallcarboxylat der allgemeinen Formel (III), worin R für Wasserstoff oder einen aliphatischen Rest mit 1 bis 5, insbesondere für einen aliphatischen Rest mit 1 bis 4 Kohlenstoffatomen steht, einsetzt.

8. Verfahren nach einem oder mehreren der Ansprüche 1 bis 7, dadurch gekennzeichnet, daß man ein Alkalimetallcarboxylat, worin Me für Na oder K, insbesondere Na steht, einsetzt.

9. Verfahren nach einem oder mehreren der Ansprüche 1 bis 8, dadurch gekennzeichnet, daß man das Chlorcarbonsäureanilid und das Alkalimetallcarboxylat im Molverhältnis 1:1 bis 1:5, insbesondere 1:1 bis 1:2 einsetzt.

10. Verfahren nach einem oder mehreren der Ansprüche 1 bis 9, dadurch gekennzeichnet, daß man Toluol, o-Xylol, m-Xylol, p-Xylol, Gemische isomerer Xylole, Ethylbenzol, Mesitylen, Chlorbenzol, Dichlorbenzol, Chlortoluol, Cyclohexan, Cumol, Decalin oder ein Gemisch dieser Lösungsmittel als inertes Lösungsmittel einsetzt.

11. Verfahren nach einem oder mehreren der Ansprüche 1 bis 10, dadurch gekennzeichnet, daß man eine aliphatische Carbonsäure mit 1 bis 6, insbesondere 2 bis 5 Kohlenstoffatomen als Carbonsäure einsetzt.

12. Verfahren nach einem oder mehreren der Ansprüche 1 bis 11, dadurch gekennzeichnet, daß man Essigsäure oder Propionsäure als Carbonsäure einsetzt.

13. Verfahren nach einem oder mehreren der Ansprüche 1 bis 12, dadurch gekennzeichnet, daß man das Alkalimetallcarboxylat und die Carbonsäure im Molverhältnis 1:0,5 bis 1:5, insbesondere 1:0,7 bis 1:2 einsetzt.

14. Verfahren nach einem oder mehreren der Ansprüche 1 bis 13, dadurch gekennzeichnet, daß man inertes Lösungsmittel zu Carbonsäure im Gewichtsverhältnis 1:0,02 bis 1:0,25, insbesondere 1:0,03 bis 1:0,15, bevorzugt 1:0,04 bis 1:0,1 einsetzt.

15. Verfahren nach einem oder mehreren der Ansprüche 1 bis 14, dadurch gekennzeichnet, daß man die Umsetzung bei 70 bis 170°C durchführt.

16. Verfahren nach einem oder mehreren der Ansprüche 1 bis 15, dadurch gekennzeichnet, daß man die Umsetzung bei 90 bis 140°C durchführt.

## Claims

1. A process for the preparation of O-acyloxycarboxanilides of the formula (I) 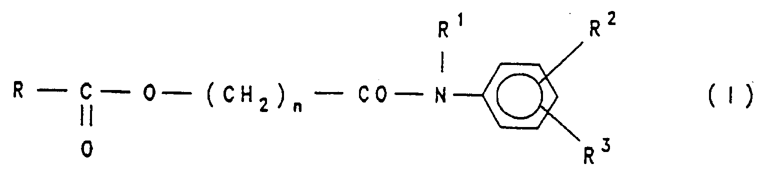 in which R is hydrogen or a radical having 1 to 6 carbon atoms, n is an integer from 1 to 10, R¹ is hydrogen or an alkyl radical having 1 to 12 carbon atoms, R² and R³ are identical or different and are hydrogen, an alkyl radical having 1 to 12 carbon atoms, an aryl radical having 6 to 12 carbon atoms, a cycloalkyl radical having 5 to 12 carbon atoms in the ring, an alkenyl or alkynyl radical having 3 to 12 carbon atoms, or NO₂, F, Cl, Br or CN, which comprises reacting a chlorocarboxanilide of the formula (II) 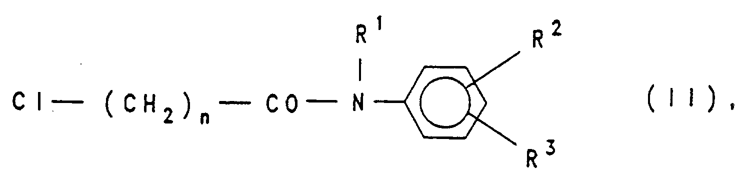 in which n, R¹, R² and R³ have the abovementioned meaning, with an alkali metal carboxylate of the formula (III)
R-COOMe (III),
in which R has the abovementioned meaning and Me is an alkali metal, in the presence of an inert solvent and of a carboxylic acid having 1 to 6 carbon atoms, at 50 to 200°C.

2. The process as claimed in claim 1, wherein a chlorocarboxanilide of the formula (II) is employed in which n is an integer from 1 to 4, in particular 1 to 2.

3. The process as claimed in claim 1 or 2, wherein a chlorocarboxanilide is employed in which n is equal to 1.

4. The process as claimed in one or more of claims 1 to 3, wherein a chlorocarboxanilide is employed in which R¹ is hydrogen or an alkyl radical having 1 to 4 carbon atoms.

5. The process as claimed in one or more of claims 1 to 4, wherein a chlorocarboxanilide is employed in which R² and R³ are identical or different and are hydrogen, an alkyl radical having 1 to 4 carbon atoms, or NO₂, F, Cl, Br or CN.

6. The process as claimed in one or more of claims 1 to 5, wherein a chlorocarboxanilide is employed in which R² is hydrogen and R³ is an alkyl radical having 1 to 4 carbon atoms, or NO₂, F, Cl, Br or CN.

7. The process as claimed in one or more of claims 1 to 6, wherein an alkali metal carboxylate of the formula (III) is employed in which R is hydrogen or an aliphatic radical having 1 to 5, in particular an aliphatic radical having 1 to 4, carbon atoms.

8. The process as claimed in one or more of claims 1 to 7, wherein an alkali metal carboxylate is employed in which Me is Na or K, in particular Na.

9. The process as claimed in one or more of claims 1 to 8, wherein the chlorocarboxanilide and the alkali metal carboxylate are employed in the molar ratio 1:1 to 1:5, in particular 1:1 to 1:2.

10. The process as claimed in one or more of claims 1 to 9, wherein toluene, o-xylene, m-xylene, p-xylene, mixtures of isomeric xylenes, ethylbenzene, mesitylene, chlorobenzene, dichlorobenzene, chlorotoluene, cyclohexane, cumene, decalin or a mixture of these solvents is/are employed as inert solvent.

11. The process as claimed in one or more of claims 1 to 10, wherein an aliphatic carboxylic acid having 1 to 6, in particular 2 to 5, carbon atoms is employed as carboxylic acid.

12. The process as claimed in one or more of claims 1 to 11, wherein acetic acid or propionic acid is employed as carboxylic acid.

13. The process as claimed in one or more of claims 1 to 12, wherein the alkali metal carboxylate and the carboxylic acid are employed in the molar ratio 1:0.5 to 1:5, in particular 1:0.7 to 1:2.

14. The process as claimed in one or more of claims 1 to 13, wherein inert solvent to carboxylic acid is employed in the weight ratio 1:0.02 to 1:0.25, in particular 1:0.03 to 1:0.15, preferably 1:0.04 to 1:0.1.

15. The process as claimed in one or more of claims 1 to 14, wherein the reaction is carried out at 70 to 170°C.

16. The process as claimed in one or more of claims 1 to 15, wherein the reaction is carried out at 90 to 140°C.

## Revendications

1. Procédé de préparation d'anilides d'acide O-acyloxycarboxylique de formule générale (I) 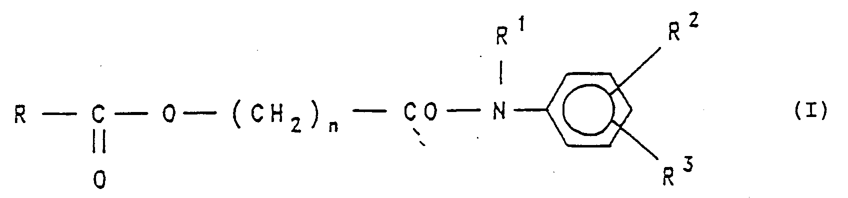 dans laquelle R est l'hydrogène ou un reste avec 1 à 6 atomes de carbone, n est un nombre entier de 1 à 10, R¹ est l'hydrogène ou un reste alkyle avec 1 à 12 atomes de carbone, R² et R³ sont identiques ou différents et représentent l'hydrogène, un reste alkyle avec 1 à 12 atomes de carbone, un reste aryle avec 6 à 12 atomes de carbone, un reste cycloalkyle avec 5 à 12 atomes de carbone dans le cycle, un reste alcényle ou alcynyle avec 3 à 12 atomes de carbone, ainsi que NO₂, F, CI, Br ou CN, caractérisé en ce que l'on fait réagir un anilide d'acide chlorocarboxylique de formule générale (II) 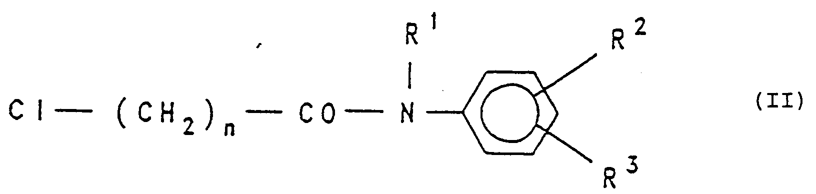 dans laquelle n, R¹, R² et R³ ont la signification donnée ci-dessus, avec un carboxylate de métaux alcalins de formule générale (III)
R-COOMe (III)
dans laquelle R a la signification donnée ci-dessus et Me représente un métal alcalin, en présence d'un solvant inerte et d'un acide carboxylique avec 1 à 6 atomes de carbone, à une température de 50 à 200 °C.

2. Procédé selon la revendication 1, caractérisé en ce que l'on utilise un anilide d'acide chlorocarboxylique de formule (II) dans laquelle n est un nombre entier de 1 à 4, plus particulièrement de 1 à 2.

3. Procédé selon la revendication 1 ou 2, caractérisé en ce que l'on utilise un anilide d'acide chlorocarboxylique dans lequel n est égal à 1.

4. Procédé selon une ou plusieurs des revendications 1 à 3, caractérisé en ce que l'on utilise un anilide d'acide chlorocarboxylique où R¹ représente l'hydrogène ou un reste alkyle avec 1 à 4 atomes de carbone.

5. Procédé selon une ou plusieurs des revendications 1 à 4, caractérisé en ce que l'on utilise un anilide d'acide chlorocarboxylique, où R² et R³ sont identiques ou différents et représentent l'hydrogène, un reste alkyle avec 1 à 4 atomes de carbone, NO₂, F, Cl, Br ou CN.

6. Procédé selon une ou plusieurs des revendications 1 à 5, caractérisé en ce que l'on utilise un anilide d'acide chlorocarboxylique où R² est l'hydrogène et R³ est un reste alkyle avec 1 à 4 atomes de carbone, NO₂, F, Cl, Br ou CN.

7. Procédé selon une ou plusieurs des revendications 1 à 6, caractérisé en ce que l'on utilise un carboxylate de métal alcalin de formule générale (III) dans laquelle R est l'hydrogène ou un reste aliphatique avec 1 à 5, plus particulièrement un reste aliphatique avec 1 à 4 atomes de carbone.

8. Procédé selon une ou plusieurs des revendications 1 à 7, caractérisé en ce que l'on utilise un carboxylate de métaux alcalins dans lequel Me représente Na ou K, plus particulièrement Na.

9. Procédé selon une ou plusieurs des revendications 1 à 8, caractérisé en ce que l'on utilise l'anilide d'acide chlorocarboxylique et le carboxylate de métal alcalin dans un rapport molaire de 1:1 à 1:5, plus particulièrement de 1:1 à 1:2.

10. Procédé selon une ou plusieurs des revendications 1 à 9, caractérisé en ce que l'on utilise en tant que solvant inerte le toluène, l'o-xylène, le m-xylène, le p-xylène, des mélanges de xylènes isomères, l'éthylbenzène, le mésitylène, le chlorobenzène, le dichlorobenzène, le chlorotoluène, le cyclohexane, le cumène, la décaline ou un mélange de ces solvants.

11. Procédé selon une ou plusieurs des revendications 1 à 10, caractérisé en ce que l'on utilise un acide carboxylique aliphatique avec 1 à 6 plus particulièrement avec 2 à 5 atomes de carbone en tant qu'acide carboxylique.

12. Procédé selon une ou plusieurs des revendications 1 à 11, caractérisé en ce que l'on utilise l'acide acétique ou l'acide propionique en tant qu'acide carboxylique.

13. Procédé selon une ou plusieurs des revendications 1 à 12, caractérisé en ce que l'on utilise le carboxylate de métal alcalin et l'acide carboxylique dans un rapport molaire de 1:0,5 à 1:5, plus particulièrement de 1:0,7 à 1:2.

14. Procédé selon une ou plusieurs des revendications 1 à 13, caractérisé en ce que l'on utilise le solvant inerte et l'acide carboxylique dans un rapport pondéral de 1:0,02 à 1:0,25, plus particulièrement de 1:0,03 à 1:0,15, de préférence de 1:0,04 à 1:0,1.

15. Procédé selon une ou plusieurs des revendications 1 à 14, caractérisé en ce que l'on met en oeuvre la réaction à une température de 70 à 170 °C.

16. Procédé selon une ou plusieurs des revendications 1 à 15, caractérisé en ce que l'on met en oeuvre la réaction à une température de 90 à 140 °C.
